# FASCICULE DE BREVET EUROPEEN

(11) **EP 4 090 395 B1**
(45) Date de publication et mention de la délivrance du brevet: **22.04.2026**
(21) Numéro de dépôt: 21701072.7
(22) Date de dépôt: 14.01.2021
(51) Int. Cl.: A61M 1/02

(54) **SYSTÈME POUR L'ÉLIMINATION SÉLECTIVE D'UNE SUBSTANCE CIBLE DANS UN FLUIDE BIOLOGIQUE**
SYSTEM ZUR SELEKTIVEN ENTFERNUNG EINER ZIELSUBSTANZ AUS EINER BIOLOGISCHEN FLÜSSIGKEIT
SYSTEM FOR SELECTIVE REMOVAL OF A TARGET SUBSTANCE FROM A BIOLOGICAL FLUID

(30) Priorité: 14.01.2020 FR 2000316
(43) Date de publication de la demande: 23.11.2022
(73) Titulaire: Maco Pharma, 59420 Mouvaux (FR)
(72) Inventeur: BREBANT, Quentin, 59370 Mons en Baroeul (FR); HOUSSIN, Timothée, 59420 Mouvaux (FR); PAQUET, Louis, 59700 Marcq en Baroeul (FR); NICOLAS, Rosa, 76560 Oherville (FR)
(74) Mandataire: Sayettat, Julien Christian
(86) Numéro de dépôt international: PCT/EP2021/050728
(87) Numéro de publication internationale: WO 2021/144380

(56) Documents cités:
- WO-A1-2011/061533
- US-A1- 2012 219 633

## Description

La présente invention concerne un système pour traiter un fluide biologique tel que le sang ou un composant sanguin par élimination sélective d'une substance cible, ainsi qu'un procédé pour traiter un fluide biologique à l'aide d'un tel système.

Elle s'applique typiquement au traitement des fluides biologiques et notamment le sang, les composants du sang ou les produits dérivés du sang destinés à être transfusés à un patient et dans le cas où la substance cible comprend les virus, les protéines telles que les protéines prion, les bactéries, les parasites, les cellules telles que les leucocytes, les cellules tumorales ou cancéreuses, les toxines, les antigènes surfaciques ou circulants , les anticorps, ou les substances endogènes ou exogènes telles que les substances exogènes utilisées dans un procédé d'inactivation des pathogènes. Elle s'applique plus particulièrement à l'élimination sélective des anticorps anti-A et/ou anti-B du sang ou du plasma.

On connaît du document WO2007/042644 une unité de filtration pour l'élimination sélective d'une substance cible présente dans le sang comprenant des particules présentant une affinité pour la substance cible. Ces particules sphériques ont en particulier un diamètre moyen compris entre 20 et 150 µm et sont maintenues dans l'unité de filtration entre deux couches de non-tissé présentant un diamètre de pore moyen inférieur à 8 µm.

Dans le document US7700746, il est décrit une colonne de filtration utilisée pour réduire la quantité d'anticorps anti-A et anti-B dans du sang total ou un plasma sanguin. La colonne comprend notamment des particules liées par l'intermédiaire d'un espaceur, à un saccharide tel qu'un déterminant du groupe sanguin A ou B. Pour la filtration du sang, les particules ont une taille comprise entre 100 et 250 µm et sont maintenues dans la colonne à l'aide d'une membrane de porosité comprise entre 30 et 100 µm.

Dans ce type de dispositif à particules, il est important de s'assurer que les particules ne contaminent pas le fluide filtré. Les dispositifs décrits ci-dessus sont appropriés pour purifier le sang à l'aide de particules ayant une taille calibrée supérieure à environ 20 µm, mais ne sont pas adaptés lorsque la taille moyenne des particules est inférieure à environ 15 µm ou encore lorsque la distribution de la taille des particules est telle qu'une proportion non négligeable de particules, par exemple 10%, possède une taille inférieure à 15 µm. L'utilisation d'un matériau de plus faible porosité pour contraindre ces petites particules dans de tels dispositifs risque d'entraver le bon écoulement du fluide au travers de tels dispositifs et/ou d'altérer la qualité du fluide biologique en retenant des composants d'intérêt.

Dans le document US 2012/0219633, il est décrit un système pour éliminer de manière successive les immunoglobulines et les leucocytes du sang. Le système comprend une poche contenant des particules se liant aux immunoglobulines ainsi qu'un filtre poreux et fibreux pour la rétention des leucocytes. Les particules sont retenues par un maillage ou un sac à tamis, c'est-à-dire un matériau à larges ouvertures, formé d'un réseau de fils métalliques ou plastiques, similaire à un filet. Par exemple, les particules sont retenues par un tamis en polyéthylène avec des ouvertures de 35-40 µm. Les ouvertures de ces tamis sont trop larges pour pouvoir retenir les leucocytes.

L'invention propose un système pour l'élimination sélective d'une substance cible présente dans le sang ou un composant sanguin à l'aide de particules pouvant être de petite taille, notamment inférieure à 15 µm, permettant de traiter dans un temps raisonnable des volumes de sang pouvant aller jusqu'à deux litres, en conservant les propriétés biologiques du sang tout en s'assurant que le sang traité est exempt de particules.

A cet effet et selon un premier aspect, l'invention propose un système pour traiter un fluide biologique tel que le sang ou un composant sanguin par élimination sélective d'une substance cible, comprenant une poche de traitement munie d'au moins un orifice d'entrée et d'au moins un orifice de sortie, ladite poche de traitement contenant un ensemble de particules présentant une affinité pour la substance cible, ledit ensemble comprenant des petites particules ayant une taille inférieure à 15 µm, le système comprenant en outre un moyen barrière pour isoler lesdites particules du fluide biologique, ledit moyen barrière étant composé d'au moins une membrane poreuse réalisée dans un matériau hydrophile, les pores de ladite membrane étant calibrés à une taille apte à empêcher le passage desdites petites particules, à savoir inférieure à 3 µm.

Selon un deuxième aspect, l'invention porte sur un procédé pour traiter un fluide biologique tel que le sang ou un composant sanguin par élimination sélective d'une substance cible à l'aide d'un système selon le premier aspect, ledit système comprenant en outre une poche de transfert en communication fluidique ou destinée à être mise en communication fluidique avec la poche de traitement par l'intermédiaire d'une tubulure de transfert, ledit procédé comprenant :
- l'introduction du fluide biologique à traiter dans la poche de traitement contenant l'ensemble de particules ayant une affinité pour ladite substance cible,
- la mise en contact dudit fluide biologique avec ledit ensemble de particules pendant un temps suffisant pour lier la substance cible auxdites particules, de sorte à obtenir un mélange de fluide biologique traité dont la substance cible a été éliminée et de particules liées ou non à la substance cible,
- le transfert dudit mélange de la poche de traitement vers la poche de transfert par l'intermédiaire de la tubulure de transfert, le moyen barrière dudit système empêchant ledit ensemble de particules de passer dans la poche de transfert, de sorte à obtenir le fluide biologique ainsi traité séparé dudit ensemble de particules, et
- le recueil du fluide biologique ainsi traité et séparé de l'ensemble de particules dans la poche de transfert 7.

Les dessins annexés illustrent l'invention :
[Fig.1] représente de façon schématique une poche de traitement d'un système selon une réalisation de l'invention dans laquelle le moyen barrière est arrangé librement dans ladite poche de traitement.
[Fig. 2] représente de façon schématique une poche de traitement d'un système selon une autre réalisation de l'invention dans laquelle le moyen barrière est fixé dans ladite poche de traitement.
[Fig. 3] représente de façon schématique un système selon une réalisation particulière comprenant la poche de traitement de la figure 1 et une poche de transfert.
[Fig. 4] représente de façon schématique un système selon une autre réalisation de l'invention comprenant une poche de traitement, une poche de transfert et un moyen barrière arrangé dans une unité filtrante disposée entre ladite poche de traitement et ladite poche de transfert.

Selon un premier aspect, l'invention propose un système pour traiter un fluide biologique par élimination d'une substance cible. Le fluide biologique est notamment le sang ou un composant sanguin destiné à être transfusé. Le composant sanguin est en particulier un concentré de globules rouges, un plasma riche en plaquettes, un plasma pauvre en plaquettes ou un concentré plaquettaire.

Il est nécessaire dans certains cas d'éliminer une substance cible du sang ou d'un composant sanguin avant sa transfusion à un patient.

Par exemple, la substance cible comprend les leucocytes, les protéines prion, les virus, les bactéries, les parasites, les champignons ou autres agents pathogènes.

Dans un autre exemple, la substance cible est un anticorps anti-A et/ou un anticorps anti-B. L'élimination des anticorps anti-A et anti-B du plasma d'un donneur permet de le rendre compatible avec les patients de n'importe quel groupe sanguin.

Dans un autre exemple encore, la substance cible est une substance utilisée pour inactiver les pathogènes du sang ou d'un composant sanguin. Une telle substance est par exemple le bleu de méthylène, un dérivé de psoralène ou la riboflavine.

L'élimination de la substance cible est totale ou partielle, c'est-à-dire suffisante pour supprimer ou réduire le risque infectieux lié aux agents infectieux, et/ou supprimer ou réduire la toxicité des substances cibles, à un niveau acceptable pour la transfusion.

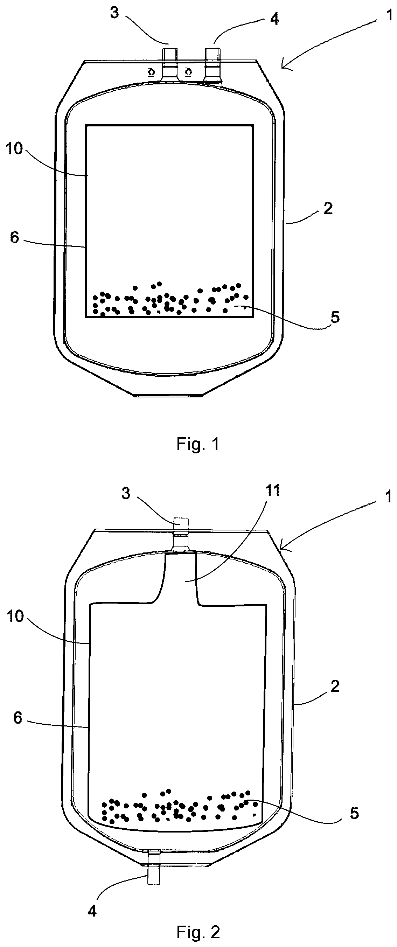

En référence aux dessins des figures 1 à 4, le système 1 comprend une poche de traitement 2 munie d'au moins un orifice d'entrée 3 et d'au moins un orifice de sortie 4. Cette poche de traitement 2 est destinée à contenir le fluide biologique à traiter.

La poche de traitement est réalisée à partir de deux feuilles souples assemblées entre elles sur leur périphérie afin de former un volume intérieur. Le volume intérieur est suffisant pour contenir le fluide à traiter. Par exemple, la poche de traitement est configurée pour contenir une quantité de fluide biologique comprise entre 20 ml et 2000 ml, notamment entre 100 ml et 700 ml.

La poche de traitement contient un ensemble de particules 5 présentant une affinité pour la substance cible. L'élimination de la substance cible est ainsi réalisée par l'intermédiaire des particules qui sont capables de se lier par affinité à la substance cible. Les particules liées à la substance cible sont ensuite séparées du fluide biologique.

Par exemple, les particules ont une affinité pour un type de substance cible. En variante, les particules ont une affinité pour plusieurs substances cibles pour permettre l'élimination simultanée de plusieurs substances cibles d'un fluide biologique.

Dans un exemple particulier, les particules comprennent un mélange de particules ayant une affinité pour les anticorps anti-A et de particules ayant une affinité pour les anticorps anti-B.

Les particules sont notamment des particules adsorbantes, telles que des particules de charbon actif, d'oxyde d'aluminium, de silice. En variante, les particules sont à base de polymère comme le polystyrène, le polycarbonate, la cellulose, le dextrane, le polyméthacrylate ou le polyacrylate.

En particulier, ces particules sont traitées physiquement et/ou chimiquement pour améliorer leur spécificité et/ou leur affinité pour la ou les substance(s) cible(s).

Plus particulièrement, les particules sont greffées avec des oligosaccharides, tels que des oligosaccharides capables de lier des anticorps tels que les anticorps anti-A et/ou les anticorps anti-B. Par exemple, ces particules sont des particules de cellulose ultrafines greffées à l'aide d'un oligosaccharide antigénique de groupe A ou B, comme celles décrites dans le document WO2016/177967. Dans un autre exemple, les particules sont celles décrites dans le document EP3141558A1 ou les billes IsoClear commercialisées par Prometic Bioseparations.

L'ensemble des particules contenues dans la poche de traitement comprend des petites particules ayant une taille inférieure à 15 µm.

Dans un premier exemple, toutes les particules sont des petites particules ayant une taille moyenne inférieure à 15 µm ; plus particulièrement inférieure à 10 µm.

Dans un autre exemple, les particules ont une taille moyenne supérieure à 15 µm, mais une partie des particules sont de petite taille, à savoir inférieure à 15 µm, notamment inférieure à 10 µm.

L'ensemble des particules peut être caractérisé par la distribution de la taille de particules. Cette mesure de la distribution de la taille des particules est notamment réalisée à l'aide d'un granulomètre par diffraction laser.

En particulier, l'ensemble des particules comprend 10% des particules avec une taille inférieure à 15 µm, notamment inférieure à 10 µm.

Les particules sont généralement substantiellement sphériques et leur taille correspond au diamètre de la sphère. Dans le cas où les particules ne sont pas sphériques, leur taille correspond au diamètre équivalent, c'est-à-dire au diamètre de la sphère de même volume qui se comporterait de manière identique à celle de la particule non sphérique lors de sa rétention.

Dans un exemple particulier, les particules sont de forme ellipsoïdale avec des dimensions approximatives de 2,2 µm par 8 µm. On considère que ces particules ont une taille d'environ 2 µm.

L'ensemble des particules 5 est généralement laissé libre dans la poche de traitement. Dans une variante, afin d'éviter leur agglomération, l'ensemble des particules est imprégné ou intégré dans une couche textile telle qu'un non-tissé, telle qu'envisagé dans le document WO2007/042644.

Selon une réalisation particulière, la quantité de particules varie de 1 à 10 mg/ml de fluide biologique, notamment de 2 à 5 mg/ml de fluide biologique.

Selon l'invention, le système comprend en outre un moyen barrière 6 qui barre le passage des particules mais permet le passage du fluide biologique.

Ce moyen barrière 6 est composé d'au moins une membrane poreuse réalisée dans un matériau hydrophile, les pores de ladite membrane étant calibrés à une taille apte à empêcher le passage desdites particules, à savoir inférieure à 3 µm.

Cette membrane poreuse est plane et possède une structure non réticulée, notamment sans enchevêtrement de fils, fibres ou brins, ni de structure en filet. Cette membrane poreuse est par exemple fabriquée par inversion de phase.

Les pores de la membrane ont une taille apte à empêcher le passage desdites particules, c'est-à-dire que la taille des pores de la membrane est substantiellement inférieure à la taille minimale des particules.

En particulier, la membrane poreuse possède une épaisseur comprise entre 90 µm et 150 µm.

Dans une réalisation particulière, pour s'assurer que l'ensemble des particules ne comprend pas de particules ayant une taille plus petite que celle des pores de la membranes poreuse, l'ensemble des particules est passé à travers un tamis ayant des pores avec une taille correspondant à celle des pores de la membrane poreuse. Les particules qui ne sont pas passées à travers du tamis forment l'ensemble de particules du système pour traiter le fluide biologique.

Les pores de la membrane sont en particulier calibrés à une taille inférieure ou égale à 3 µm, en particulier inférieure ou égale à 1 µm. Par exemple, les pores de la membrane sont d'environ 0,65 µm.

Cette membrane de porosité d'environ 0,65 µm est avantageuse en ce qu'elle permet en outre de retenir simultanément les constituants cellulaires tels que les leucocytes du plasma sanguin.

La membrane est réalisée dans un matériau hydrophile choisi parmi les matériaux naturellement hydrophiles ou les matériaux, à base de matière plastique, rendus hydrophiles. En effet, le caractère hydrophile du matériau est important pour assurer une bonne hémocompatibilité avec le sang ou les composants sanguins.

Par exemple, le matériau hydrophile est choisi parmi les polymères et/ou copolymères à base de polypropylène, de polyester, de polyamide, de polyéthylène haute ou basse densité, de polyuréthane, le poly(fluorure de vinylidène) ou les produits à base de cellulose tel que l'acétate de cellulose et ses dérivés.

Ces produits polymériques ne sont généralement pas hydrophiles naturellement et doivent être traités par des méthodes physiques ou chimiques, pour leur conférer lesdites propriétés hydrophiles.

Ces traitements consistent par exemple dans le greffage de substituants hydrophiles, par exemple des groupements de type hydroxyle ou carboxylique, sur le polymère, selon des méthodes connues. Un autre traitement consiste à effectuer un traitement plasma gazeux, notamment oxygène.

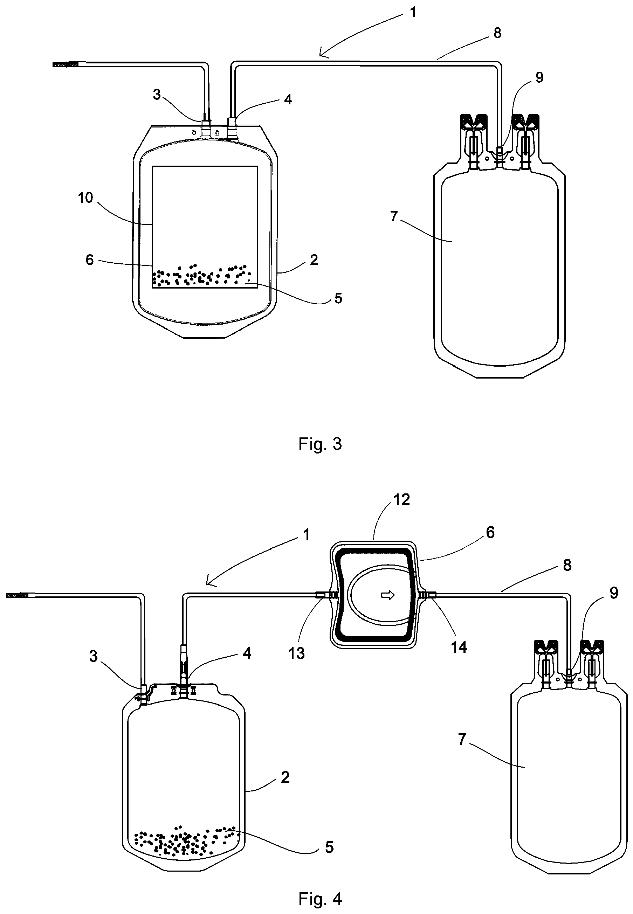

En relation avec les figures 3 et 4, le système comprend en outre une poche de transfert 7 en communication fluidique ou destinée à être mise en communication fluidique avec la poche de traitement 2 par l'intermédiaire d'une tubulure de transfert 8. Cette poche de transfert 7 est destinée à recueillir le fluide biologique traité, appauvri en substance cible et dépourvu de particules.

La tubulure de transfert 8 est reliée à l'orifice de sortie 4 de la poche de traitement 2 et à un orifice d'entrée 9 de la poche de transfert 7.

Le moyen barrière 6 est disposé à l'intérieur ou à l'extérieur de la poche de traitement 2.

Dans une réalisation représentée sur les figures 1 à 3, le moyen barrière 6 est disposé à l'intérieur de la poche de traitement 2. Dans ce cas, le moyen barrière est destiné à retenir les particules dans la poche de traitement 2. Lors du transfert du fluide biologique traité de la poche de traitement 2 dans la poche de transfert 7, les particules 5 sont confinées dans la poche de traitement 2. Le fluide biologique recueilli dans la poche de transfert 7 est ainsi dépourvu de particules.

Dans une première configuration représentée sur la figure 1, le moyen barrière 6 est sous forme d'un sachet 10 renfermant les particules 5, ledit sachet étant composé de ladite membrane poreuse.

La porosité de la membrane poreuse formant le sachet est telle que les particules 5 ne peuvent sortir du sachet 10 en traversant ses parois, mais le fluide biologique peut la traverser pour être mis en contact avec ces particules 5.

Le sachet est réalisé par exemple en assemblant sur les côtés une membrane poreuse mise en double ou bien assemblant sur le fond et les côtés deux membranes mises l'une au-dessus de l'autre. L'assemblage est réalisé notamment par soudure thermique, haute fréquence ou ultrasons.

Dans une variante, le sachet 10 est un sachet à double paroi, chacune des parois étant composée de la membrane poreuse. Dans cette variante, le sachet à double parois est par exemple produit en arrangeant un premier sachet contenant les particules dans un second sachet, chacun des premier et deuxième sachets étant composé de la membrane poreuse. Ainsi, si des petites particules sortent du premier sachet, elles sont retenues par la membrane poreuse du second sachet.

Dans une autre variante, le sachet 10 comprend un premier et un deuxième joint de soudure périphérique à distance l'un de l'autre. Ainsi, si l'un des joints de soudure périphérique est défectueux, l'autre joint de soudure périphérique assure l'étanchéité du sachet 10.

Afin de s'assurer du bon confinement des particules dans le sachet 10, le sachet est un sachet à double paroi et à double joint de soudure.

Sur la figure 1, le sachet 10 est scellé, c'est-à-dire que le haut du sachet est fermé, notamment par soudage. Les particules sont alors enfermées à l'intérieur du sachet 10.

Le sachet 10 est avantageusement libre dans la poche de traitement 2. En variante, il est fixé par l'un de ses côtés à l'un des bords de la poche de traitement 2.

Dans une variante de configuration représentée sur la figure 2, le sachet 10 contenant les particules 5 est agencé à l'intérieur de la poche de traitement 2 et présente une ouverture 11, ladite ouverture étant en communication fluidique avec l'orifice d'entrée 3 de la poche de traitement 2.

Dans cette configuration, le fluide biologique à traiter est introduit dans le sachet 10 de la poche de traitement 2 pour être mis en contact avec les particules, puis passe au travers de la membrane poreuse formant le sachet.

Dans une autre configuration non représentée, le moyen barrière 6 est sous forme d'une paroi composée de ladite membrane poreuse, ladite paroi étant arrangée à l'intérieur de la poche de traitement 2 de sorte à délimiter un compartiment d'entrée en communication fluidique avec l'orifice d'entrée 3 de la poche de traitement et un compartiment de sortie en communication fluidique avec l'orifice de sortie 4 de la poche de traitement 2. Le compartiment d'entrée de la poche de traitement 2 renferme les particules 5.

Avantageusement, la paroi comprenant la membrane poreuse est maintenue dans un cadre étanche, ce qui facilite l'assemblage du moyen barrière 6 dans la poche de traitement 2.

Dans une autre réalisation illustrée sur la figure 4, le moyen barrière 6 est disposé à l'extérieur de la poche de traitement 2, sur le chemin d'écoulement défini par la tubulure de transfert 8.

Le moyen barrière 6, extérieur à la poche de traitement 2, est par exemple sous forme d'une unité filtrante 12, ladite unité filtrante renfermant un milieu de filtration composé d'au moins ladite membrane poreuse.

Dans cette réalisation, le système comprend une poche de transfert 7 en communication fluidique avec la poche de traitement 2 par l'intermédiaire d'une tubulure de transfert 8, ladite unité filtrante 12 étant arrangée sur ladite tubulure de transfert 8.

Dans ce cas, lors du transfert du fluide biologique de la poche de traitement 2 dans la poche de transfert 7, les particules 5 sont retenues par le moyen barrière 6 sans atteindre la poche de transfert 7. Ainsi, le fluide biologique traité recueilli dans la poche de transfert 7 est dépourvu de particules.

L'unité filtrante 12 comporte une enveloppe souple, rigide ou semi-rigide, à l'intérieur de laquelle la membrane poreuse formant moyen barrière 6 est disposée. L'unité filtrante comprend en outre un orifice d'entrée 13 et un orifice de sortie 14, le milieu de filtration délimitant un compartiment d'entrée en communication avec l'orifice d'entrée 13 et un compartiment de sortie en communication avec l'orifice de sortie 14.

Le milieu de filtration est avantageusement maintenu dans l'unité filtrante par l'intermédiaire d'un cadre souple et étanche.

Le milieu de filtration est composé uniquement d'une ou plusieurs membranes poreuses.

Avantageusement, le milieu de filtration comprend en outre une ou plusieurs couches d'un matériau poreux hydrophile afin d'éviter le colmatage de la membrane poreuse et/ou afin de filtrer d'autres substances du fluide biologique.

Encore plus avantageusement, le milieu de filtration est apte à éliminer les leucocytes du sang ou d'un composant sanguin.

En particulier, le milieu de filtration comprend en outre en amont de la ou les membranes poreuses, une ou plusieurs couches d'un matériau poreux hydrophile présentant chacune une taille moyenne de pore supérieure à la taille des pores de la membrane poreuse.

En particulier, le matériau poreux hydrophile est un non-tissé filé-lié ou de fibre soufflées à l'état fondu.

Par exemple, le milieu de filtration comprend en amont de la membrane poreuse, plusieurs couches de non-tissé ayant une porosité moyenne comprise entre 8 et 12 µm.

Une telle unité filtrante est avantageusement adaptée à éliminer en outre les constituants cellulaires tels que les leucocytes, du plasma sanguin. Un exemple particulier d'une telle unité filtrante est décrite dans le document EP 0 953 361 A1.

Dans le cas où l'ensemble de particules 5 lient les anticorps anti-A et/ou anti-B du plasma sanguin, le système comportant une telle membrane poreuse trouve une application pour obtenir un plasma universel déleucocyté destiné à être transfusé.

Selon un deuxième aspect, on décrit maintenant un procédé pour traiter un fluide biologique tel que le sang ou un composant sanguin par élimination sélective d'une substance cible à l'aide d'un système selon le premier aspect de l'invention. Le système comprenant en outre une poche de transfert 7 en communication fluidique avec la poche de traitement par l'intermédiaire d'une tubulure de transfert 8.

Le procédé comprend :
- l'introduction du fluide biologique à traiter dans la poche de traitement 2 contenant l'ensemble de particules 5 ayant une affinité pour ladite substance cible,
- la mise en contact dudit fluide biologique avec ledit ensemble de particules 5 pendant un temps suffisant pour lier la substance cible auxdites particules, de sorte à obtenir un mélange de fluide biologique traité dont la substance cible a été éliminée et de particules liées ou non à la substance cible,
- le transfert dudit mélange de la poche de traitement 2 vers la poche de transfert 7 par l'intermédiaire de la tubulure de transfert 8, le moyen barrière 6 dudit système empêchant l'ensemble de particules de passer dans la poche de transfert, de sorte à obtenir le fluide biologique ainsi traité séparé dudit ensemble de particules, et
- le recueil du fluide biologique ainsi traité et séparé de l'ensemble de particules dans la poche de transfert.

Le fluide biologique est notamment du sang préalablement prélevé d'un donneur ou un composant sanguin issu de sang prélevé d'un donneur et séparé par exemple par filtration et/ou centrifugation des autres composants sanguins.

Notamment, dans le cas où l'ensemble des particules présente une affinité pour les anticorps anti-A et/ou anti-B, le procédé est utilisé pour obtenir un plasma universel sans particule, prêt à être transfusé.

Exemple préliminaire : rétention des particules par une membrane poreuse

Afin de tester la capacité d'une membrane poreuse selon l'invention à retenir des petites particules, le test suivant a été mené.

Une unité filtrante a été réalisée comprenant un milieu de filtration constitué d'amont en aval de : une couche de non-tissé filé lié en polyester, quatre couches de non-tissé en polypropylène melt-blown ayant chacune une masse surfacique d'environ 40 g/m² et une perméabilité à l'air d'environ 110 l/m²/s, une couche de membrane en poly(fluorure de vinylidène) ayant subi un traitement hydrophile et ayant une porosité de 0,65µm, une couche de non-tissé en polypropylène melt-blown ayant une masse surfacique d'environ 40 g/m² et une perméabilité à l'air d'environ 110 l/m²/, et une couche de non-tissé filé lié en polyester. La surface de filtration est d'environ 20 cm².

Une poche a été remplie avec une suspension de 250 ml d'eau ppi et de particules de cellulose ultrafine (5 mg/ml), de forme ellipsoïdale ayant une taille approximative de 2,2 µm x 8 µm.

Le contenu de la poche a été passé au travers de la poche filtrante et le nombre de particules après filtration a été déterminé à l'aide d'un compteur de particules dans les liquides.

### [Tableau 1]

**Tableau 1**

| | Volume d'eau analysé | Nombre de particules moyen > 5 µm | Nombre de particules moyen > 10 µm | Nombre de particules moyen > 25 µm |
|---|---|---|---|---|
| Poche | 120 ml | 87,86 par ml | 7,64par ml | 0,08 par ml |

Ces résultats sont conformes à la pharmacopée américaine (American Pharmacopea, Volume 6, <788> Particulate matter in injections, USP 42, edition 2019, 6942-6946) et à la pharmacopée européenne (European Pharmacopeia 10.0, Section < 2.9.19 > Particulate contamination : Sub-Visible particles, 360-362, 04/2011 : 20919) qui stipulent que pour des préparations injectables ayant un volume supérieur à 100 ml, les conditions suivantes doivent être respectées :

### [Tableau 2]

**Tableau 2**

| Volume de la préparation parentérale | Taille des particules | Nombre de particules par ml Pharmacopée US | Nombre de particules par ml Pharmacopée EP |
|---|---|---|---|
| > 100 ml | ≥ 10 µm | 25 par ml | 12 par ml |
| | ≥ 25 µm | 3 par ml | 2 par ml |

### Exemple : élimination des anticorps anti-B du plasma sanguin de groupe O

### Matériel

750 mg de particules de cellulose ultrafine greffées avec un oligosaccharide antigénique de groupe B, de forme ellipsoïdale ayant une taille approximative de 2,2 µm x 8 µm ont été introduites dans une poche en PVC.

La poche filtrante utilisée dans cet exemple est identique à celle décrite dans l'exemple préliminaire ci-dessus.

### Traitement du plasma

Environ 250 ml de plasma de groupe O a été obtenu par centrifugation d'une unité de sang total de groupe O (environ 500 ml). Après introduction du plasma dans la poche en PVC contenant les particules, la poche PVC a été agitée horizontalement pendant environ 30 minutes puis son contenu a été filtré par gravité au travers de la poche filtrante.

### Analyses

Les titres en anticorps de type immunoglobuline G (IgG) et immunoglobuline M (IgM) ont été déterminés dans le plasma initial, c'est-à-dire avant traitement et dans le plasma final, c'est-à-dire après la filtration au travers de la poche filtrante.

La détermination du titre en anticorps est réalisée par un test d'agglutination direct pour le titrage des IgM et indirect pour le titrage des IgG.

Des échantillons de plasma (initial et final) sont dilués en tampon PBS, par dilution successive d'un facteur 2 (1/1, 1/2,..,, 1/ 64) et mis en contact avec des hématies du groupe sanguin B. Le titre rendu correspond à la dernière dilution où les agglutinats de globules rouges dus à la présence d'anticorps sanguins restent visibles.

Les résultats sont montrés ci-dessous.

### [Tableau 3]

**Tableau 3**

| Ti tr e | Titre anticorps initial | Titre anticorps final | Gain de titration |
|---|---|---|---|
| Ig M | 1/128 | 1/2 | 6 |
| Ig G | 1/64 | 1/2 | 5 |

Des dosages biochimiques de facteurs plasmatiques ont également été menés. Les résultats sont montrés dans le tableau 4 ci-dessous.

### [Tableau 4]

**Tableau 4**

| | Plasma non traité | Plasma après filtration |
|---|---|---|
| Facteur Vc | 58% | 57% |
| Facteur VIIIc | 31% | 30% |
| Facteur XIc | 85% | 78% |
| fibrinogène | 2,91 g/l | 2,78 g/l |
| prothrombine | 79% | 79% |

Dans ce tableau, les concentrations en facteurs de coagulation facteur V, facteur VIII, facteur IX et prothrombine sont exprimées en pourcentage par rapport à un plasma humain normal étalon. Les dosages sont des dosages chronométriques.

La filtration n'a pas d'impact sur les facteurs plasmatiques testés :
prothrombine, fibrinogène, Facteur V, Facteur VIII et Facteur XI.

## Revendications

1. Système (1) pour traiter un fluide biologique tel que le sang ou un composant sanguin par élimination sélective d'une substance cible, comprenant une poche de traitement (2) munie d'au moins un orifice d'entrée (3) et d'au moins un orifice de sortie (4), ladite poche de traitement (2) contenant un ensemble de particules (5) présentant une affinité pour la substance cible, ledit ensemble comprenant des petites particules ayant une taille inférieure à 15 µm, **caractérisé en ce qu'**il comprend en outre un moyen barrière (6) composé d'au moins une membrane poreuse réalisée dans un matériau hydrophile, les pores de ladite membrane étant calibrés à une taille apte à empêcher le passage desdites petites particules, à savoir inférieure à 3 µm.

2. Système selon la revendication 1, **caractérisé en ce que** le matériau hydrophile est choisi parmi les polymères et/ou copolymères à base de polypropylène, de polyester, de polyamide, de polyéthylène haute ou basse densité, de polyuréthane, le poly(fluorure de vinylidène) ou les produits à base de cellulose tel que l'acétate de cellulose et ses dérivés.

3. Système selon l'une des revendications 1 ou 2,
**caractérisé en ce qu'**il comprend en outre une poche de transfert (7) en communication fluidique ou destinée à être mise en communication fluidique avec la poche de traitement (2) par l'intermédiaire d'une tubulure de transfert (8).

4. Système selon l'une quelconque des revendications 1 à 3, **caractérisé en ce que** le moyen barrière (6) est disposé à l'intérieur de la poche de traitement (2).

5. Système selon la revendication 4, **caractérisé en ce que** le moyen barrière (6) est sous forme d'un sachet (10) renfermant ledit ensemble de particules (5), ledit sachet (10) étant composé de ladite membrane poreuse.

6. Système selon la revendication 5, **caractérisé en ce que** le sachet (10) est un sachet à double paroi, chacune des parois étant composée de la membrane poreuse.

7. Système selon l'une des revendications 5 ou 6,
**caractérisé en ce que** le sachet (10) est scellé ou présente une ouverture (11), ladite ouverture (11) étant en communication fluidique avec l'orifice d'entrée (3) de la poche de traitement (2).

8. Système selon la revendication 4, **caractérisé en ce que** le moyen barrière (6) est sous forme d'une paroi composée de ladite membrane poreuse, ladite paroi étant arrangée à l'intérieur de la poche de traitement (2) de sorte à délimiter un compartiment d'entrée en communication fluidique avec l'orifice d'entrée (3) de la poche de traitement (2) et un compartiment de sortie en communication fluidique avec l'orifice de sortie (4) de la poche de traitement (2), ledit compartiment d'entrée de la poche de traitement (2) renfermant ledit ensemble de particules (5).

9. Système selon la revendication 8, **caractérisé en ce que** la paroi comprenant la membrane poreuse est maintenue dans un cadre étanche.

10. Système selon l'une des revendications 1 ou 2,
**caractérisé en ce que** le moyen barrière (6) est sous forme d'une unité filtrante (12), ladite unité filtrante renfermant un milieu de filtration composé d'au moins ladite membrane poreuse.

11. Système selon la revendication 10, **caractérisé en ce qu'**il comprend en outre une poche de transfert (7) en communication fluidique avec la poche de traitement (2) par l'intermédiaire d'une tubulure de transfert (8), ladite unité filtrante (12) étant arrangée sur la tubulure de transfert (8).

12. Système selon l'une des revendications 10 ou 11,
**caractérisé en ce que** le milieu de filtration comprend en outre une ou plusieurs couches d'un matériau poreux hydrophile présentant chacune une taille moyenne de pore supérieure à la taille moyenne de pore de la membrane poreuse.

13. Système selon la revendication 12, **caractérisé en ce que** le matériau poreux hydrophile est un non-tissé filé-lié ou de fibre soufflées à l'état fondu.

14. Système selon l'une quelconque des revendications 10 à 13, **caractérisé en ce que** milieu de filtration est apte à éliminer les leucocytes du sang ou d'un composant sanguin.

15. Procédé pour traiter un fluide biologique tel que le sang ou un composant sanguin par élimination sélective d'une substance cible à l'aide d'un système (1) selon l'une quelconque des revendications 3 à 14 comprenant :
- l'introduction du fluide biologique à traiter dans la poche de traitement (2) contenant l'ensemble de particules (5) ayant une affinité pour ladite substance cible,
- la mise en contact dudit fluide biologique avec ledit ensemble de particules (5) pendant un temps suffisant pour lier la substance cible auxdites particules, de sorte à obtenir un mélange de fluide biologique traité dont la substance cible a été éliminée et de particules liées ou non à la substance cible,
- le transfert dudit mélange de la poche de traitement (2) vers la poche de transfert (7) par l'intermédiaire de la tubulure de transfert (8), le moyen barrière (6) dudit système empêchant ledit ensemble de particules de passer dans la poche de transfert (7), de sorte à obtenir le fluide biologique ainsi traité séparé dudit ensemble de particules, et
- le recueil du fluide biologique ainsi traité et séparé de l'ensemble de particules dans la poche de transfert (7).

## Patentansprüche

1. System (1) zum Behandeln einer biologischen Flüssigkeit, wie beispielsweise Blut oder eines Blutbestandteils durch selektives Entfernen einer Zielsubstanz, umfassend einen Behandlungsbeutel (2), der mit mindestens einer Einlassöffnung (3) und mindestens einer Auslassöffnung (4) versehen ist, wobei der Behandlungsbeutel (2) eine Anordnung von Partikeln (5) enthält, die eine Affinität für die Zielsubstanz aufweisen, die Anordnung umfassend kleine Partikel, die eine Größe von weniger als 15 µm aufweisen, **dadurch gekennzeichnet, dass** er ferner eine Barriereeinrichtung (6) umfasst, das aus mindestens einer porösen Membran besteht, die aus einem hydrophilen Material gefertigt ist, wobei die Poren der Membran auf eine Größe kalibriert sind, die geeignet ist, um den Durchgang der kleinen Partikel, nämlich kleiner als 3 µm, zu verhindern.

2. System nach Anspruch 1, **dadurch gekennzeichnet, dass** das hydrophile Material ausgewählt ist aus Polymeren und/oder Copolymeren auf Basis von Polypropylen, Polyester, Polyamid, Polyethylen hoher oder niedriger Dichte, Polyurethan, Poly(vinylidenfluorid) oder Produkten auf Basis von Cellulose, wie beispielsweise Celluloseacetat und seinen Derivaten.

3. System nach einem der Ansprüche 1 oder 2, **dadurch gekennzeichnet, dass** es ferner einen Transferbeutel (7) umfasst, der über einen Transferschlauch (8) in Flüssigkeitsverbindung mit dem Behandlungsbeutel (2) ist oder dazu bestimmt ist, in Flüssigkeitsverbindung damit gebracht zu werden.

4. System nach einem der Ansprüche 1 bis 3, **dadurch gekennzeichnet, dass** die Barriereeinrichtung (6) im Inneren des Behandlungsbeutels (2) angeordnet ist.

5. System nach Anspruch 4, **dadurch gekennzeichnet, dass** die Barriereeinrichtung (6) in Form eines Beutels (10) ist, der die Anordnung von Partikeln (5) umschließt, wobei der Beutel (10) aus der porösen Membran besteht.

6. System nach Anspruch 5, **dadurch gekennzeichnet, dass** der Beutel (10) ein doppelwandiger Beutel ist, wobei jede der Wände aus der porösen Membran besteht.

7. System nach einem der Ansprüche 5 oder 6, **dadurch gekennzeichnet, dass** der Beutel (10) versiegelt ist oder eine Öffnung (11) aufweist, wobei die Öffnung (11) in Flüssigkeitsverbindung mit der Einlassöffnung (3) des Behandlungsbeutels (2) ist.

8. System nach Anspruch 4, **dadurch gekennzeichnet, dass** die Barriereeinrichtung (6) in Form einer Wand ist, die aus der porösen Membran besteht, wobei die Wand innerhalb des Behandlungsbeutels (2) eingerichtet ist, um eine Einlasskammer in Flüssigkeitsverbindung mit der Einlassöffnung (3) des Behandlungsbeutels (2) und eine Auslasskammer in Flüssigkeitsverbindung mit der Auslassöffnung (4) des Behandlungsbeutels (2) zu begrenzen, wobei die Einlasskammer des Behandlungsbeutels (2) die Anordnung von Partikeln (5) umschließt.

9. System nach Anspruch 8, **dadurch gekennzeichnet, dass** die Wand, die die poröse Membran umfasst, in einem dichten Rahmen gehalten wird.

10. System nach einem der Ansprüche 1 oder 2, **dadurch gekennzeichnet, dass** die Barriereeinrichtung (6) in Form einer Filtereinheit (12) ist, wobei die Filtereinheit ein Filtrationsmedium umschließt, das aus mindestens der porösen Membran besteht.

11. System nach Anspruch 10, **dadurch gekennzeichnet, dass** es ferner einen Transferbeutel (7) umfasst, der über einen Transferschlauch (8) in Flüssigkeitsverbindung mit dem Behandlungsbeutel (2) ist, wobei die Filtereinheit (12) an dem Transferschlauch (8) eingerichtet ist.

12. System nach einem der Ansprüche 10 oder 11, **dadurch gekennzeichnet, dass** das Filtrationsmedium ferner eine oder mehrere Schichten aus einem hydrophilen porösen Material umfasst, die jeweils eine mittlere Porengröße aufweisen, die größer ist als die mittlere Porengröße der porösen Membran.

13. System nach Anspruch 12, **dadurch gekennzeichnet, dass** das hydrophile poröse Material ein Spinnvlies oder eine schmelzgeblasene Faser ist.

14. System nach einem der Ansprüche 10 bis 13, **dadurch gekennzeichnet, dass** das Filtrationsmedium geeignet ist, um Leukozyten aus Blut oder einem Blutbestandteil zu entfernen.

15. Verfahren zum Behandeln einer biologischen Flüssigkeit, wie beispielsweise Blut oder eines Blutbestandteils durch selektives Entfernen einer Zielsubstanz mittels eines Systems (1) nach einem der Ansprüche 3 bis 14, umfassend:
- Einleiten der zu behandelnden biologischen Flüssigkeit in den Behandlungsbeutel (2), der die Anordnung von Partikeln (5) enthält, die eine Affinität für die genannte Zielsubstanz aufweisen,
- Inkontaktbringen der biologischen Flüssigkeit mit der Anordnung von Partikeln (5) über eine Zeit, die ausreichend ist, um die Zielsubstanz an die Partikel zu binden, um ein Gemisch aus behandelter biologischer Flüssigkeit, aus dem die Zielsubstanz entfernt worden ist, und Partikeln, die an die Zielsubstanz gebunden sind oder nicht, zu erlangen,
- Transferieren des Gemischs aus dem Behandlungsbeutel (2) über den Transferschlauch (8) in den Transferbeutel (7), wobei die Barriereeinrichtung (6) des Systems verhindert, dass die Anordnung von Partikeln in den Transferbeutel (7) gelangt, um die somit behandelte biologische Flüssigkeit getrennt von der Anordnung von Partikeln zu erlangen, und
- Auffangen der somit behandelten und von der Anordnung von Partikeln getrennten biologischen Flüssigkeit in dem Transferbeutel (7).

## Claims

1. A system (1) for treating a biological fluid such as blood or a blood component by selective removal of a target substance, comprising a treatment bag (2) provided with at least one inlet orifice (3) and at least one outlet orifice (4), said treatment bag (2) containing a set of particles (5) having an affinity for the target substance, said set comprising small particles having a size of less than 15 µm, **characterised in that** the system further comprises a barrier means (6) composed of at least one porous membrane made of a hydrophilic material, the pores of said membrane being calibrated to a size suitable for preventing the passage of said small particles, namely less than 3 µm.

2. The system according to claim 1, **characterised in that** the hydrophilic material is selected from polymers and/or copolymers based on polypropylene, polyester, polyamide, high- or low-density polyethylene, polyurethane, polyvinylidene fluoride, or cellulose-based products such as cellulose acetate and its derivatives.

3. The system according to one of claims 1 to 2, **characterised in that** it further comprises a transfer bag (7) in fluid communication or intended to be put in fluid communication with the treatment bag (2) via a transfer tubing (8).

4. The system according to any one of claims 1 to 3, **characterised in that** the barrier means (6) is disposed inside the treatment bag (2).

5. The system according to claim 4, **characterised in that** the barrier means (6) is in the form of a pouch (10) enclosing said set of particles (5), said pouch (10) being composed of said porous membrane.

6. The system according to claim 5, **characterised in that** the pouch (10) is a double-walled pouch, each of the walls being composed of the porous membrane.

7. The system according to any one of claims 5 to 6, **characterised in that** the pouch (10) is sealed or has an opening (11), said opening (11) being in fluid communication with the inlet orifice (3) of the treatment bag (2).

8. The system according to claim 4, **characterised in that** the barrier means (6) is in the form of a wall composed of said porous membrane, said wall being arranged within the treatment bag (2) so as to delimit an inlet compartment in fluid communication with the inlet orifice (3) of the treatment bag (2) and an outlet compartment in fluid communication with the outlet orifice (4) of the treatment bag (2), said inlet compartment of the treatment bag (2) enclosing said set of particles (5).

9. The system according to claim 8, **characterised in that** the wall comprising the porous membrane is held in a sealed frame.

10. The system according to one of claims 1 or 2, **characterised in that** the barrier means (6) is in the form of a filter unit (12), said filter unit enclosing a filtration medium composed of at least said porous membrane.

11. The system according to claim 10, **characterised in that** it further comprises a transfer bag (7) in fluid communication with the treatment bag (2) via a transfer tubing (8), said filter unit (12) being arranged on the transfer tubing (8).

12. The system according to one of claims 10 or 11, **characterised in that** the filtration medium further comprises one or more layers of a hydrophilic porous material each having an average pore size greater than the average pore size of the porous membrane.

13. The system according to claim 12, **characterised in that** the hydrophilic porous material is a spunbonded non-woven fabric or a melt-blown fibre.

14. The system according to any one of claims 10 to 13, **characterised in that** the filtration medium is able to remove leukocytes from blood or a blood component.

15. A method for treating a biological fluid such as blood or a blood component by selective removal of a target substance using a system (1) according to any of claims 3 to 14, comprising the steps of:
- introducing the biological fluid to be treated into the treatment bag (2) containing the set of particles (5) having an affinity for said target substance,
- contacting said biological fluid with said set of particles (5) for a time sufficient to bind the target substance to said particles, so as to obtain a mixture of treated biological fluid from which the target substance has been removed and particles bound or not bound to the target substance,
- transferring said mixture from the treatment bag (2) to the transfer bag (7) via the transfer tubing (8), the barrier means (6) of said system preventing said set of particles from passing into the transfer bag (7), so as to obtain the biological fluid thus treated separated from said set of particles, and
- collecting the biological fluid thus treated and separated from the set of particles in the transfer bag (7).
